# EUROPEAN PATENT APPLICATION

(11) **EP 2 017 586 A1**
(43) Date of publication of application: **21.01.2009**
(21) Application number: 07014291.4
(22) Date of filing: 20.07.2007
(51) Int. Cl.: G01F 1/34, A61B 5/087, A61M 16/00, A61M 16/08

(54) **Monitor for CPAP/Ventilator apparatus**

(71) Applicant: MAP Medizin-Technologie GmbH, 82152 Martinsried (DE)
(72) Inventor: Martin, Dion Charles Chewe, Concord, New South Wales 2137 (AU); Schätzl, Stefan, 82362 Weilheim (DE)
(74) Representative: Vossius & Partner

(57) **Abstract**

A monitor (200) includes a measurement line segment (202) adapted to be coupled between a patient interface and a positive airway pressure device. The measurement line segment defines a gas flow path. A sensor (208) includes two ports (210) in communication with the gas flow path through the measurement line segment, and a recording device (203) is adapted to record data collected by the sensor.

## Description

### CROSS-REFERENCE TO RELATED APPLICATION

PCT Publication No. WO 2006/056444, published June 1, 2006, and its priority application (German Application No. 10 2004 056 748.4, filed November 24, 2004), are each incorporated herein by reference in its entirety.

### FIELD OF THE INVENTION

The invention relates to a monitor for surveying measured values that are indicative of a person's breathing, e.g., for use with a CPAP/ventilator apparatus.

### BACKGROUND OF THE INVENTION

Based on Sullivan's discovery that sleep-related breathing problems (e.g., due to airway constrictions that occur during the sleeping phase and to obstructive airway constrictions) can be treated by administering breathing gas, especially ambient air, at an elevated pressure level to the patient's airways, devices for administering this breathing gas have accordingly been developed since the 1980s.

Pumping the breathing gas up to this elevated pressure level is predominantly done, in the devices used in practice, by rpm-regulated blowers. These blowers, unlike bulky pumping devices, have a pressure lock through which ambient air can flow into a system segment that is at elevated pressure and can flow back through this lock again during an expiration phase.

The delivery of the pumped breathing gas to a patient is typically done via a flexible breathing gas line and a patient interface, such as a mask. The breathing gas line and the patient interface form part of the system segment that is at elevated breathing gas pressure. In this region, a derivation of CO₂ from the exhaled breathing gas can be achieved by forming defined leakage openings, in the region of the overpressure system segment near the patient, for scavenging this segment.

Alternatively, respiratory insufficiency and respiratory failure are treated with a variety of modes (e.g., pressure-targeted, volume-targeted, combinations of pressure and volume) and a variety of interfaces (e.g., mask, endotracheal tube, tracheostomy tube), up to 24 hours/day.

For adapting the pumping capacity of the blower or regulating the breathing gas pressure, numerous pressure regulation concepts are known. For instance, it is possible in particular to regulate the pumping capacity such that over the entire breathing cycle, largely constant static pressures in the region of the mask are obtained. It is also known to regulate the breathing gas pressure such that during an expiration phase, for instance, the breathing gas pressure is lowered, to lessen the breathing work the patient must do. Devices are also known by which an automatic analysis of the patient's breathing is done continuously, based on software, and the breathing gas pressure is done largely in real time on the basis of this automatic analysis of the instantaneous breathing.

In the diagnosis and/or treatment of sleep-related breathing problems, the use of different devices and device components can cause difficulties in assessing the need for treatment and the success of treatment, and in defining suitable device settings.

### SUMMARY

One aspect of the invention is directed to an apparatus or method for making an improved assessment, in terms of its conclusiveness, reliability and/or applicability, of a patient's breathing in the course of a treatment phase or a diagnosis.

According to one embodiment of the invention, a monitor is provided for surveying signals indicative of a patient's breathing and/or breathing device parameters. The monitor has a housing structure (1) defining a gas flow path; a measurement line segment (2) provided to or in the vicinity of the housing structure (1) and structured to be in communication with a breathing gas line segment; a sensor provided along the gas flow path to generate a signal indicative of the breathing gas flow; and an electronic recording device (3) to record one or more signals indicative of the breathing gas flow, and/or information derived from the one or more signals. According to an embodiment, the monitor comprises a resistor, particularly for causing a pressure differential Δp, and a transducer for transducing a sensed pressure differential Δp into a voltage. In an embodiment, such resistor is adapted to be located in the gas flow path whereas the transducer is adapted to be located in the housing of the monitor. Among the recorded signals are the pressure of the breathing gas flow and/or the flow rate of the breathing gas flow.

It thus becomes advantageously possible, in treating a patient by using a CPAP device or other ventilator device, for instance, to record and assess the quality of treatment in a neutral and standardized way.

The monitor may be embodied as an autonomous recording module.
Thus, it can be incorporated into typical breathing gas tubing systems, and, in particular, can be plugged into them. In an embodiment, the monitor may have its own power supply, which may be in the form of a battery device or a rechargeable battery device.

The breathing system may be the vented, single-limbed (bi-directional) circuit typical of CPAP, BiPAP or general ventilation systems, or may be a dual-limbed circuit more typical of critical care ventilators, with a flow/pressure sensor configured in each of the two limbs (one for inspiratory flow, and another for expiratory flow).

The monitor may include an electronic recording device, e.g., in the form of a memory card or a flash stick. The monitor may be in the form of a module. Further, the monitor can be used in conjunction with a feedback loop to assist with real-time control of a flow generator (e.g., CPAP) or other ventilator. It is possible to provide an interface device on the module, for transmitting the detected signals to an evaluation or monitoring computer system. The interface device may be embodied as analog signals, as a USB interface, as a network interface, or in particular as a wireless interface. The interface device may be embodied such that the directly surveyed data and/or the data stored in memory can be read out.

It is possible to design an electronic data processor, provided in the region of the monitor, such that the data processor can be configured in a program-based way (e.g., using software) for a certain detection task or a certain detection concept. For instance, the degree of compression or a certain intermediate evaluation procedure can be defined, e.g., in a software-based manner.

The monitor may include a measurement device to survey a signal that is indicative of the breathing gas pressure prevailing at that time.

It is possible to make integral the tubular element, forming the measurement channel, in such a way that this tubular element can advantageously be cleaned and sterilized. The surveying of the flow signal can be done using structures of the kind used as such in pneumotachography equipment.

According to another embodiment, there is provided a flow generator; a patient interface; a breathing gas line segment to communicate the flow generator and the patient interface; and a monitor as described above. The monitor is in communication with the breathing line segment. In an embodiment, an antibacterial filter may be provided in order to protect the monitor from contaminations of the breathing gas.

Another aspect of the invention relates to a monitor including a measurement line segment adapted to be coupled between a patient interface and a positive airway pressure device. The measurement line segment defines a gas flow path. A flow resistor and/or sensor includes two ports in communication with the gas flow path through the measurement line segment, and a transducer and/or recording device is adapted to record data collected by the sensor.

Yet another aspect of the invention relates to a method for providing ventilation therapy including measuring differential pressure along a gas flow path between a patient interface and a positive airway pressure device, recording data derived from the differential pressure measurement, providing the recorded data to an evaluation system, evaluating the recorded data, and - where appropriate - adjusting ventilation therapy and/or prescribing an alternate therapy based on the evaluation of the recorded data.

Other aspects, features, and advantages of this invention will become apparent from the following detailed description when taken in conjunction with the accompanying drawings, which are a part of this disclosure and which illustrate, by way of example, principles of this invention.

### BRIEF DESCRIPTION OF THE DRAWINGS

Further details and characteristics are described in or apparent from the ensuing description in conjunction with the drawings, in which:

Fig. 1 is a perspective view of an embodiment of a monitor according to one embodiment of the invention;

Fig. 2 is a schematic illustration explaining the disposition of the monitor shown in Fig. 1 inside a breathing gas path;

Fig. 3 is a schematic illustration explaining an example of internal structure of the monitor shown in Fig. 1;

Fig. 4A is a schematic view of a basic structure of a monitor according to another embodiment of the present invention;

Fig. 4B is a schematic view of a basic structure of a monitor according to another embodiment of the present invention;

Figs. 5 and 6 are exemplary perspective views of two embodiments of monitors based on the basic structure of Fig. 4A;

Fig. 7 is a perspective view of a monitor including an external sensor according to another embodiment of the present invention;

Fig. 8A shows a diagram of the pressure curve of a therapy device and Fig. 8B shows a diagram of the flow curve of a therapy device for two respiration cycles; and

Fig. 9A shows a diagram of the pressure curve of a therapy device and Fig. 9B shows a diagram of the flow curve of a therapy device for a longer time period than that shown in Figs. 8A and 8B.

### DETAILED DESCRIPTION OF ILLUSTRATED EMBODIMENTS

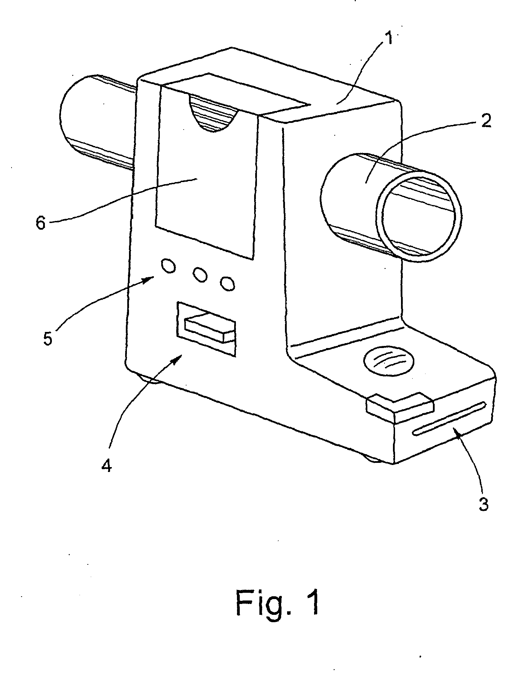

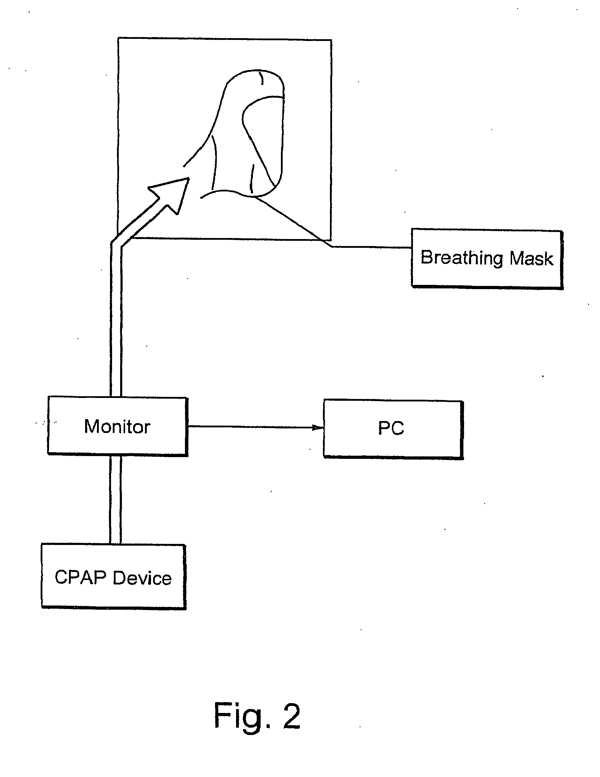

In Fig. 1, a monitor for surveying signals indicative of a patient's breathing is shown. The monitor may have a modular form. The monitor includes a housing structure 1 defining a gas flow path or a measurement section and a measurement line segment 2 that can be coupled to or otherwise in communication with a breathing gas line segment (also known as a gas delivery conduit) (see Fig. 2). The measurement line segment 2 is in communication with a sensor, e.g., a flow measuring instrument (e.g., a flow meter) to generate a signal indicative of the breathing gas flow. The sensor is in communication with the gas flow path. The monitor further includes an electronic recording device 3 for recording signals indicative of the breathing gas flow, or optionally also information derived from them. In an embodiment, the monitor is in the form of a module that is functionally, if not physically, positioned between the CPAP device (or other flow generator or positive airway pressure device) and the patient. Information obtained from and/or derived from the monitor can be used as input to the flow generator or ventilator algorithm. Thus, the monitor may generate information used in a feedback loop.

The monitor is provided with an interface device 4, which is embodied here simply as a USB port, for example. Via this interface device 4, the measurement signals surveyed in the region of the measurement line segment via the flow measuring instrument can be picked up continuously. It is also possible via the interface device for a data processor, e.g., provided in the monitor, to be configured with a view to a particular kind of data survey that is wanted.

The monitor is also equipped with a display 5, e.g., one or more LEDs, preferably of different colors. It is possible to activate the LEDs such that different colors and/or LEDs indicate whether breathing that is obscured by artifacts has been detected. In an embodiment, the respective information is displayed on an external monitor, such as on a PC monitor.

The monitor is furthermore powered with a power supply, e.g., in the form of a battery device. The battery device can be changed, once a cover device 6 (positioned over a battery chamber) has been removed. It is also possible for the power supply to be in the form of a rechargeable battery device. The charging of the rechargeable battery device can optionally be done directly via the power that can be tapped in the region of the USB port.

The monitor could also include one or more inputs from additional sensing devices, e.g., oxygen saturation from a pulse oximeter, photo-plethysmographic data from a pulse oximeter, transcutaneous CO₂ monitoring, respiratory effort monitoring, or sleep assessment systems. In doing so, the monitor exacts greater ability in assisting the managing clinician to manage the patient, for instance through the ability to infer sleep state, arousal, respiratory effort and correlate these with the respiratory pressures and flows.

Alternatively, the data acquired from the monitor may, within its associated PC application, be converged with data logged from additional sensing devices for additional specificity in managing the patient.

In an embodiment, the monitor may be designed such that the recording device, provided for recording the data indicative of the breathing gas flow, is removable from the feedback module for the sake of further signal evaluation. In this exemplary embodiment, the recording device is embodied as a memory card. It is also possible to embody the recording device as a USB flash stick, for example, and the USB flash stick can optionally be connected directly via the USB port provided here.

In an embodiment, the monitor includes a pressure detector or pressure sensor, for generating a signal indicative of the breathing gas pressure prevailing at that time. A sample rate appropriate for each individual data stream can be imposed. The breathing gas flow signals may be subjected to data compression and stored, for instance in MP3 format or in some other way, in approximated form by means of polynomial functions.

As seen in Fig. 2, the monitor may be coupled directly into a segment of the breathing gas line segment that extends between a mask and a CPAP device. The monitor could be classified as an "in-line" monitor, i.e., it is positioned along the gas delivery conduit, between the flow generator (blower) and the mask. The monitor can also be coupled directly to an evaluation circuit, in particular a PC, that is typically more powerful than the electronic circuit device provided in the monitor. It is also possible to design the monitor such that data is forwarded wirelessly, for instance using an IR interface or a Bluetooth™ interface. However, signal conversion and characteristic curve assessment may be still done in the region of the monitor, so that regardless of the measured value pickup technology used in the measurement line segment, the flow signal is readable in digital form, being linearized or defined in a standardized way.

In an embodiment, the recording concept executed by the monitor during the observation phase is configurable in a software-based manner.

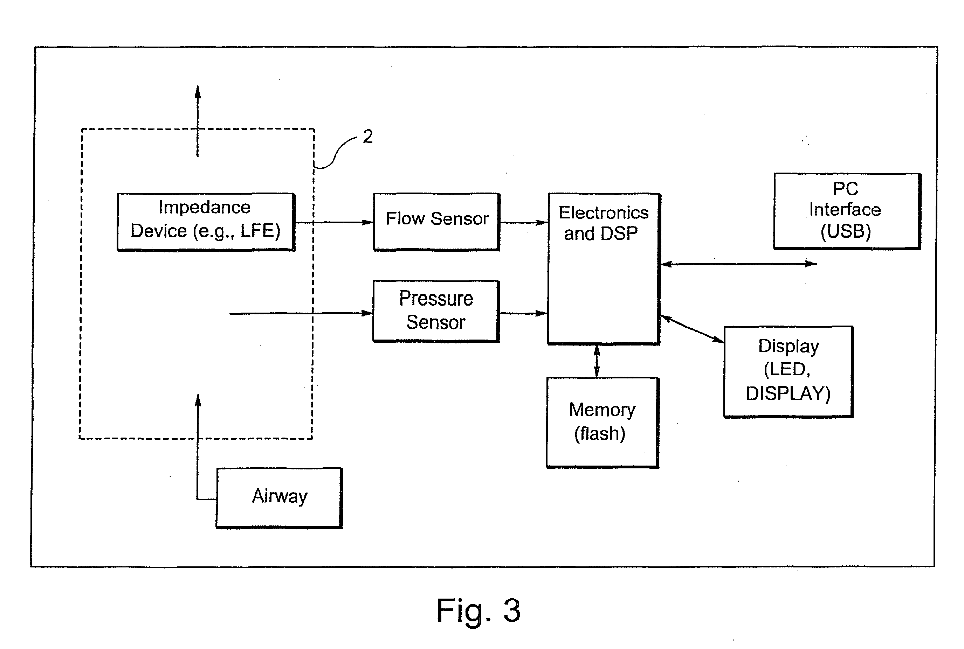

In Fig. 3, the internal structure of a monitor of an embodiment of the invention is shown schematically. Monitor includes measurement line segment 2, described above in conjunction with Fig. 1, provided with a measurement setup intended for generating a signal indicative of the breathing gas flow.

The measurement setup may be embodied as a ram pressure pickup element, flow resistor, or an impedance device that together with a differential pressure sensor across it or bypass flow around it transduces the magnitude and direction of flow (e.g., inline impedances such as an LFE (laminar flow element), fixed orifice, and variable-area orifice). Additional flow sensor possibilities include ultrasonic flow metering, hot-wire anemometry, and rotameters. The signals picked up via these corresponding measurement devices can be filtered by a filter device and forwarded to an electronic recording device (digital, programmable electronic memory) provided in the monitor. The monitor shown schematically here also includes a pressure sensor, and the signals surveyed by this pressure sensor are also forwarded to the electronic circuit. Thus, the differential pressure sensor measures Δp across the inline impedance to determine flow and the pressure sensor may be in the form of a gauge pressure sensor that reports the pressure within the air circuit relative to atmosphere.

It may also be advantageous to monitor the composition of gases within the circuit, for example oxygen concentration and carbon-dioxide concentration, for a more complete assessment of the delivered therapy and the patient's exhalate.

The data based on the measurement signals and generated by the electronic circuit device are stored in a predetermined storage pattern, e.g., on a replaceable storage medium (in this case a flash memory card). The programming of the evaluation electronics in the equipment can be done via an interface device, in particular a PC interface, such as a USB port. The monitor also includes display devices, such as LEDs or display devices. The display devices may be embodied such that with them, relatively high-quality reproduction of the results of evaluation, or also of raw data, is made possible.

The coupling of the measurement line segment 2 into a suitable breathing gas line system can be done by embodying the measurement line segment 2 such that it is compatible with hose connection cuffs that are known per se.

Fig. 3 shows one example of the basic construction of the monitor. In that portion of the gas path or airway segment defined by the measurement line segment 2, the flow can be measured via flow resistor, an impedance device, or a laminar element. The flow may be measured in both directions with the same precision. The pressure of the breathing gas in this portion of the gas path or airway segment may be also measured. This pressure is typically in the range of 0 to 80 hectopascals. The thus surveyed signals can be processed and stored in memory by the electronics. In the memory, both raw data and (preferably) evaluated events are stored. This information can be transmitted and displayed in real time via an interface, e.g., a PC interface. It is also possible, via a display, to pick up or display information directly at the monitor. The evaluation of the measurement signals surveyed with the monitor can be done in a manner known per se by the "Apnea-Link" evaluation software developed by the present Applicant.

The monitor is suitable for use not only in the clinical setting (e.g., sleep clinic, hospital ward, doctor's office), but also as a measurement system for performing standardized monitoring of therapy done at home. The monitor makes it possible to analyze and compare most of the various kinds of equipment on the market in terms of their performance, efficiency and/or effectiveness. For example, the monitor operates independently of the CPAP Device (also referred to as a flow generator or a positive airway pressure device) so that the monitor may be used for analyzing many different types of flow generator products. The monitor may also be used as part of a feedback loop, e.g., it becomes possible to collect data indicative of breathing with high resolution and to use the data for subsequent clinical studies and for developing algorithms for automatic detection of breathing problems or for automatically adapting the breathing gas pressure. In addition, PC analysis of data collected by the monitor may be adapted to different market segments, e.g., sleep disordered breathing, respiratory insufficiency, cardiology, etc.

In an embodiment, the PC software adapted to analyze data collected by the monitor may be based on ApneaLink™ software and algorithms for ResMed's ApneaLink™ device. Such ApneaLink™ software may look for periodic central apneas or abnormal breathing patterns under therapy, allowing more appropriate therapies to be prescribed. Further details regarding ApneaLink™ are provided in U.S. Patent Nos. 4,982,738 and 5,275,159, and WO 2005/23109, EP 07 10 5728.5, each of which is incorporated herein by reference in its entirety. Exemplary algorithms that may be used by the monitor are described in U.S. Patent Nos. 5,704,345, 6,029,665, 6,238,675, 6,363,933, 6,367,474, 6,502,572, 6,817,361, 6,988,498, 6,644,312, 6,845,773, 7,089,937, 6,532,959, 6,951,217, 7,077,132, 6,840,240, and 6,814,073 and PCT Application Nos. PCT/AU2004/001651, PCT/AU2005/000895, PCT/AU2004/001652, PCT/AU04/000272, PCT/US2004/019598, and PCT/AU2005/001627, each of which is incorporated herein by reference in its entirety.

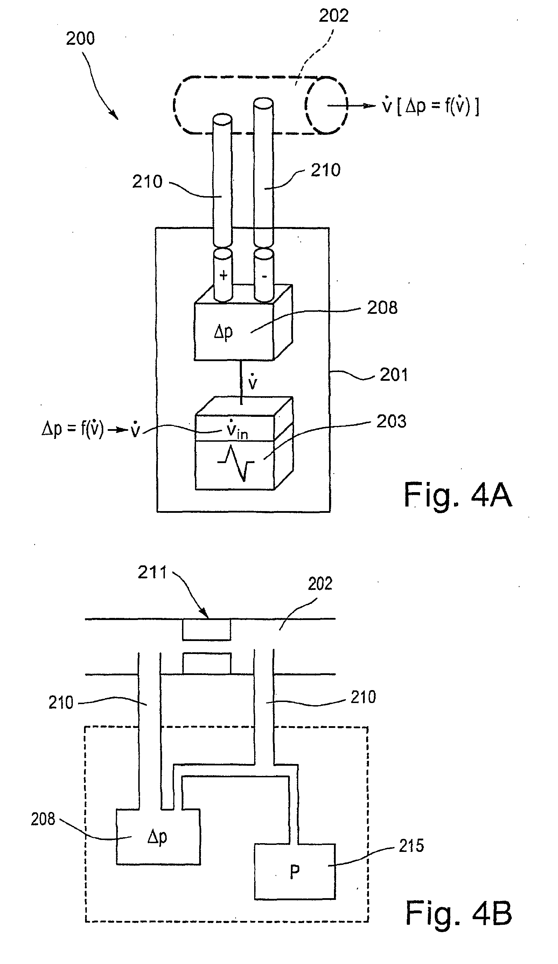

Fig. 4A is a schematic view of a monitor 200 according to an alternative embodiment of the present invention. In this embodiment, the monitor may include one or more portions of an existing ApneaLink™ device or recorder that is restructured (e.g., hardware change) so that it is adapted to measure differential pressure inside a tube system under therapy.

In the illustrated embodiment, the monitor 200 includes a housing 201, a measurement line segment 202 adapted to be coupled between a mask and a CPAP device, a differential pressure sensor 208 including two pressure ports 210 in communication with the gas flow path through the measurement line segment 202, and a processing unit including a recording device 203 adapted to record data collected by the differential pressure sensor 208. In use, the monitor 200 is coupled between a mask and a CPAP device or ventilator and measures differential pressure along the gas flow path via the two pressure ports 210. The recordings of the recording device 203 may be input into the ApneaLink™ software and analyses may be run with the recorded data.

In the illustrated embodiment, the two pressure ports 210 are positioned to determine a pressure differential Δp occurring at a flow resistor or inline impedance (e.g., a reduced flow diameter or an obstacle such as a grid) located between the two pressure ports 210. For example, Fig. 4B illustrates an inline impedance 211 between ports 210. The two ports 210 are typically distanced from one another in the direction of flow. The distance between ports and depth of the ports in the measurement line segment are sufficient to provide a reliable Δp (e.g., see also MAP's Pneumoflow™). Based on such Δp, the flow can be calculated (the gas flow is a function of Δp: [Δp = f(ν̇)]), e.g., see Fig. 4A.

Both ports 210 are connected to the differential pressure sensor 208 (or transistor - terminology may be aligned to "resistor", i.e., the part in the breathing gas flow, and "transducer", i.e., the part in the monitor transducing the Δp into voltage).

Fig. 4B illustrates an alternative embodiment in which the monitor 200 includes both a differential pressure sensor 208 (structured to measure Δp across an inline impedance 211) and a gauge pressure sensor 215 that reports the pressure within the air circuit relative to atmosphere.

In this embodiment, the monitor 200 may also serve as a flow recorder and may be used with many different types of CPAP devices or flow generators. In one example, the monitor may be used in conjunction with a relatively simple positive airway pressure device which combination results in a system with approximately the capabilities of a more sophisticated positive airway pressure device. In addition, the monitor 200 may be structured to provide similar statistics or data that the ApneaLink™ software is adapted to produce or analyze to suit the target patient population.

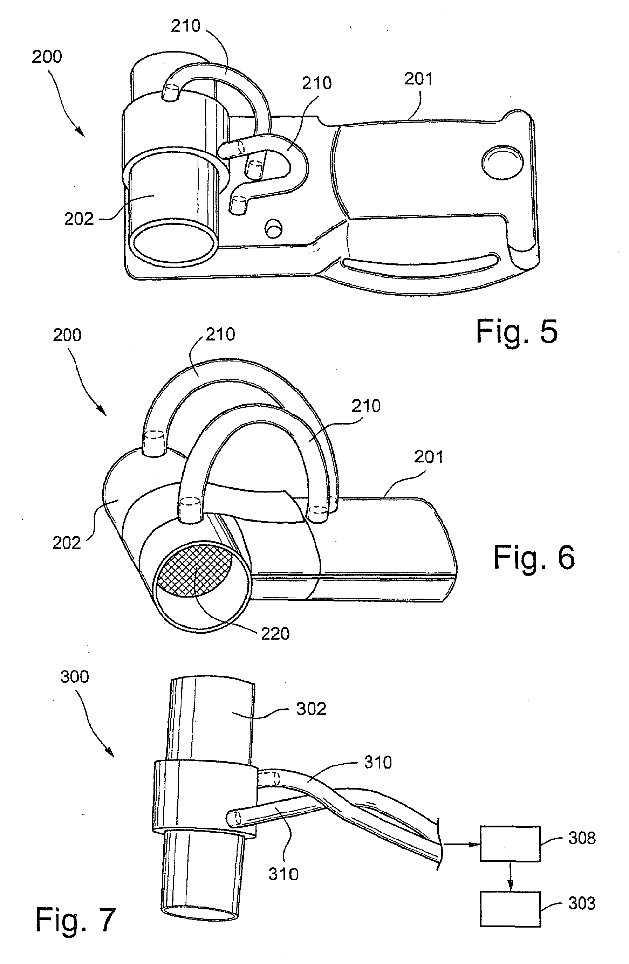

Figs. 5 and 6 illustrate exemplary embodiments of the monitor 200. As illustrated, each monitor 200 may include one or more portions based on ResMed's ApneaLink™ device or recorder, e.g., housing, internal recording device.

In Fig. 5, the monitor includes a resistor or inline impedance having a change (e.g., reduction) in the flow diameter or cross section (not shown) in order to provide Δp. This arrangement may facilitate cleaning of the monitor.

In Fig. 6, the monitor includes a resistor or inline impedance having an obstacle such as a grid 220 introduced into the flow diameter (i.e., between the ports 210) in order to provide a Δp. This arrangement may provide a more reliable measurement.

Fig. 7 illustrates a monitor 300 including an external sensor that can be connected next to a mask. As illustrated, the monitor 300 includes a measurement line segment 302 and two ports 310 in communication with the gas flow path through the measurement line segment 302. The two ports 310 are coupled to an external differential pressure sensor 308 and a processing unit including a recording device 303 may be provided that is adapted to record data collected by the sensor. The sensor and recording device are arranged external or distanced from the measurement line segment along the gas flow path, e.g., preferably close to the patient. The monitor 300 may include a resistor or inline impedance having a change (e.g., reduction) in the flow diameter or cross section such as that in Fig. 5 or an obstacle such as a grid 220 in Fig. 6.

As noted above, monitors according to embodiments of the present invention may be structured to scrutinize or analyze ventilation or CPAP therapy on any CPAP/ventilator device. For example, the monitor may be structured to sense therapy pressure and/or flow, which when correlated with SpO₂ (oxygen saturation), breathing effort, spontaneous breath-rate, arousal, and sleep time, allows holistic monitoring of CPAP and ventilation therapies applicable to any CPAP/ventilator device.

This arrangement allows increased patient monitoring in the home so that patient therapy and sleep may be improved and/or enhanced. That is, the monitor may be structured for use on all home therapy devices to help detect and interpret residual SDB problems. The associated PC software (e.g., ApneaLink™ software) may incorporate an expert system approach to troubleshooting, and in doing so, offer solutions. For example, if leak is suspected as being problematic, the system can offer solutions such as an improved mask (e.g., cross-reference information on ResMed masks), setting of synchrony controls on ResMed VPAP products, reduced pressure rise-time, etc. Alternatively, if upper-airway instability is suspected, the system can offer advice to increase end expiratory pressure (EEP) or to prescribe auto-titrating CPAP/EEP devices. Further, in cases where either periodic breathing or hypoventilation are detected, the system may recommend a device with a timed back-up ventilation mode (e.g., VPAP3 ST), or a servo-ventilator device specifically targeting such conditions (such as VPAP Adapt, AutoSet CS2, AutoVPAP, etc.). Alternatively, if the detected threats to effective therapy are sufficiently urgent (such as sustained hypoventilation, high respiratory rates, etc.), an alert may be routed to the supervising clinician via established telecommunication links (e.g., ResTraxx pager, modem, etc.).

In situations where an alternate therapy is suggested to the clinician, the monitors PC software application may offer a prescription for the alternate therapy, to further assist the managing clinician. Web-links to recommended devices may also be provided.

A general principle with one embodiment of the invention is that the companion PC application acts as an "expert system", minimizing the clinician's effort in perusing data and deducing issues, and incorporating as many different sensing modalities as are available in such deductions.

Benefits of such a system include one or more of the following:

(1) offering service that helps the physician/home healthcare dealer efficiently manage their patient;

(2) educating users regarding optional and/or optimal equipment, e.g., superior mask seal, advantageous synchrony settings, positive airway pressure device for patients whose ideal therapy may change over time (e.g., AutoSet), proposing superior therapy algorithms. Links to online ordering can further ease this process;

(3) improving patient experience and treatment, leading to improved compliance and outcome, based on both detecting problems and reducing the level of skill needed for the solution of those problems; and

(4) for therapy devices, such as ResMed therapy device, easier patient management if monitor recommendations are integrated with the therapy device's configuration software (e.g., ResScan™ data card reader), allowing seamless optimization.

In an embodiment, optimization of therapy may include monitoring the correct application of the therapy values (e.g., laid down by the medical staff) by the ventilation system (e.g., check if correct flow/pressure being provided). If therapy control is requested by law, such control will not be restricted to be performed in a lab but may also be performed at home or at any place by using a monitor according to an embodiment of the invention. The monitor according to an embodiment of the invention may even provide detection of "complex sleep apnea" (e.g., Cheyne-Stokes that may occur during therapy of OSA). Also, the monitor can work under the application of pressure, i.e., during therapy.

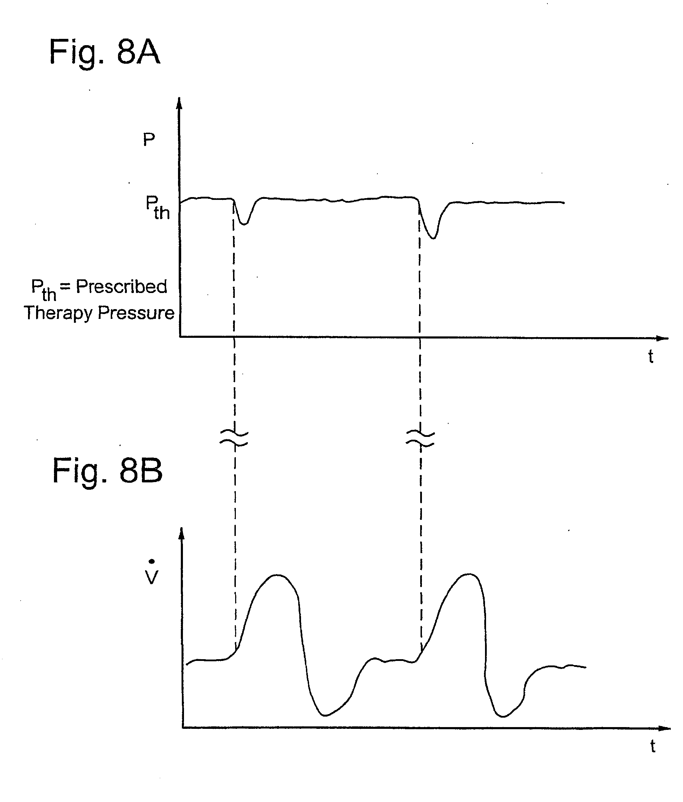

Figs. 8A to 9B illustrate exemplary data collected by a monitor according to an embodiment of the present invention. Fig. 8A shows a diagram of the pressure curve of a therapy device and Fig. 8B shows a diagram of the flow curve of a therapy device. As illustrated, each diagram shows two respiration cycles along the time axis. In these figures, the reduction in pressure as a reaction on the increase in flow, i.e., as indicated by dashed lines, is of interest (e.g., bad device performance).

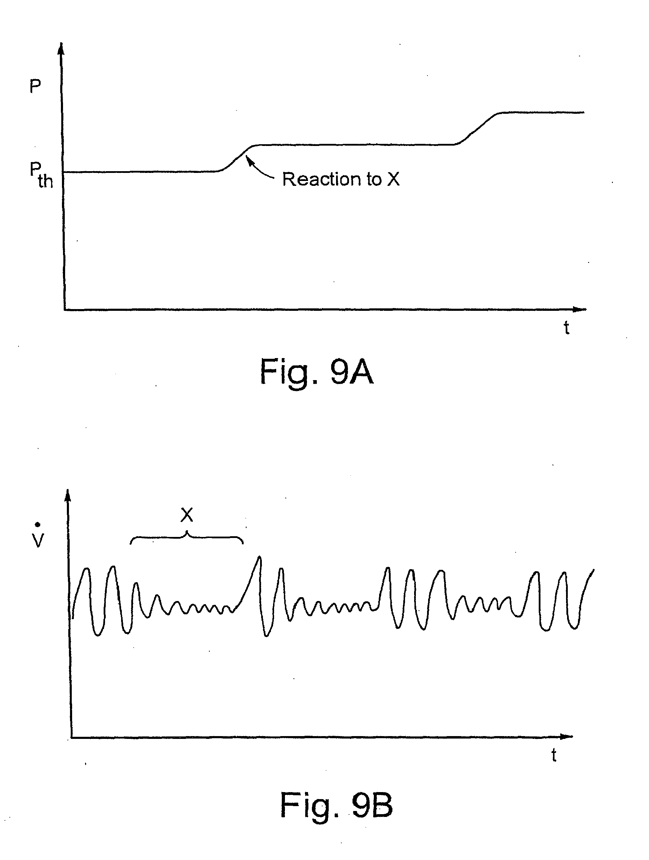

Figs. 9A and 9B illustrate pressure and flow diagrams of a therapy device, respectively, for a longer time period. These figures illustrate the reaction of the therapy device on medical features (X), e.g. increase of pressure.

In an embodiment, the monitor may be adapted to interlock with ResMed devices, e.g., monitor will not operate unless it is attached to a ResMed therapy device (e.g., powered by ResMed therapy device's auxiliary port), to optimize therapy and synergism of componentry pairings.

While the invention has been described in connection with what are presently considered to be the most practical and preferred embodiments, it is to be understood that the invention is not to be limited to the disclosed embodiments, but on the contrary, is intended to cover various modifications and equivalent arrangements included within the spirit and scope of the invention. Also, the various embodiments described above may be implemented in conjunction with other embodiments, e.g., aspects of one embodiment may be combined with aspects of another embodiment to realize yet other embodiments. In addition, while the invention has particular application to patients who suffer from sleep-disordered breathing, it is to be appreciated that patients who suffer from other illnesses (e.g., respiratory failure, respiratory insufficiency, congestive heart failure, diabetes, morbid obesity, stroke, bariatric surgery, etc.) can derive benefit from the above teachings. Moreover, the above teachings have applicability in non-medical applications.

## Claims

1. A monitor, comprising:
a measurement line segment adapted to be coupled between a patient interface and a positive airway pressure device, the measurement line segment defining a gas flow path;
a sensor including two ports in communication with the gas flow path through the measurement line segment; and
a recording device adapted to record data collected by the sensor.

2. The monitor according to claim 1, wherein the sensor includes a differential (Δp) pressure sensor.

3. The monitor according to any one of claims 1-2, wherein the measurement line segment is provided to a housing.

4. The monitor according to any one of claims 1-3, further comprising one or more additional sensors in communication with the gas flow path.

5. The monitor according to claim 4, wherein the one or more additional sensors includes a photo-plethysmography sensor, O₂ sensor, CO₂ sensor, and/or saturation sensor.

6. The monitor according to any one of claims 1-5, further comprising an alarm or alert arising from a sensed parameter collected by the sensor.

7. The monitor according to any one of claims 1-6, wherein the sensor includes dual limbs.

8. A ventilation system, comprising:
a positive airway pressure device;
a patient interface;
a breathing gas line segment to communicate the positive airway pressure device and the patient interface; and
a monitor according to any one of claims 1-3, the monitor being in communication with the breathing gas line segment.

9. The ventilation system according to claim 8, further comprising an evaluation system configured to evaluate the recorded data collected by the sensor.

10. The ventilation system according to claim 8, wherein the evaluation system includes a PC.

11. The ventilation system according to any one of claims 8-10, wherein the evaluation system is configured to provide at least one of a recommendation of alternate therapy, a prescription associated with the alternate therapy, and web-links to recommended devices.

12. The ventilation system according to any one of claims 8-11, wherein the positive airway pressure device is adapted to provide CPAP and/or BiPAP therapy.

13. The ventilation system according to any one of claims 8-12, wherein the system provides closed-loop therapy based on at least one sensed parameter.

14. A method for providing ventilation therapy, comprising:
measuring differential pressure along a gas flow path between a patient interface and a positive airway pressure device;
recording data derived from the differential pressure measurement;
providing the recorded data to an evaluation system;
evaluating the recorded data; and
where appropriate, adjusting ventilation therapy and/or prescribing an alternate therapy based on the evaluation of the recorded data.

15. The method according to claim 14, wherein the ventilation therapy includes CPAP and/or BiPAP therapy.
